# EUROPEAN PATENT SPECIFICATION

(11) **EP 0 730 036 B1**
(45) Date of publication and mention of the grant of the patent: **19.04.2000**
(21) Application number: 96101581.5
(22) Date of filing: 05.02.1996
(51) Int. Cl.: C12P 35/04, C12P 37/04, C12P 17/18

(54) **Process for the enzymatic synthesis of beta-lactam antibiotics in the presence of an enzyme inhibitor**
Verfahren zur enzymatischen Synthese von beta-laktam Antibiotika in Anwesenheit von einem Enzyminhibitor
Procédé pour la synthèse enzymatique d'antibiotiques bèta-lactame en présence d'un inhibiteur d'enzyme

(30) Priority: 28.02.1995 IT MI950383; 11.01.1996 IT MI960033
(43) Date of publication of application: 04.09.1996
(73) Proprietor: ACS DOBFAR S.p.A., 20067 Tribiano (Milano) (IT)
(72) Inventor: Zenoni, Maurizio, I-20067 Paullo, Milan (IT); Tagliani, Auro R., I-27058 Voghera, PV (IT); Cannas, Emiliano, I-20098 San Giuliano Milanese, Milan (IT); Venturelli, Angelo, I-20063 Cernusco sul Naviglio, Milan (IT)
(74) Representative: Frignoli, Luigi

(56) References cited:
- WO-A-92/01061
- WO-A-93/12250
- WO-A-95/34675

## Description

This invention relates to an improved enzymatic process for preparing penicillins and cephalosporins of formula (I) and (II) 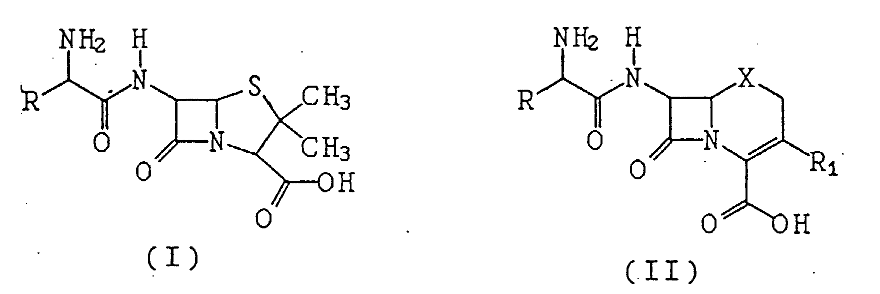 in which X is S or CH₂, R is a 6-term hydrocarbon ring optionally substituted, and R₁ is a hydrogen atom, a halogen atom, a methyl group, a methoxy group, a C₁-C₄ alkenyl group or a methylene group bound to an organic radical by an oxygen, sulphur or nitrogen atom.

US-A-3816253 describes a process for preparing penicillins or cephalosporins by reacting an α-substituted α-amino acid with a derivative of 7-aminocephalosporanic or 7-aminodesacetoxycephalosporanic acid in the presence of an active micro-organism or enzyme in aqueous solution, at a temperature of between +5°C and +50°C, and preferably between +20°C and 40°C. It has been found that by operating as described in the latter US patent, the desired final product yield is strongly reduced because of parallel concomitant reactions forming by-products which are difficult to separate from the reaction mixture.

EP-A-0473008 proposes a method for producing penicillins and cephalosporins of formula (I) or (II) which is intended to eliminate or at least reduce the aforesaid drawbacks. In this method, 6-aminopenicillanic or 7-aminocephalosporanic acid is reacted with an α-substituted α-amino acid of formula R-CH(NH₂)-COOH or a reactive derivative thereof, in the presence of an immobilized penicillin acylase, the reaction being performed in an aqueous medium at a temperature of between -5°C and +20°C, and preferably at about +4°C.

The reaction conditions are clearly stated in EP-A-0473008, which also details 25 preparation examples with very good yields (90% or more) of the desired final product.

In this respect it should be noted that a process can be implemented industrially only if the yields are sufficiently high (about 90%) and the final product is easily purified.

EP-A-0473008 mentions α-substituted α-amino acids and their reactive derivatives for use as starting substances in general terms, the only amino acid specifically mentioned being D-phenylglycine methylester.

The present applicants have performed many careful experimental tests following the procedure described in the examples given in EP-A-0473008. The results obtained have however been entirely discouraging.

In this respect, it has been verified that if in the reaction medium the molar ratio of said amino acid is less than 4 moles per mole of 6-aminopenicillanic or 7-aminocephalosporanic acid used, the yield of the desired final product is very low (less than about 60%), and as such is industrially unacceptable.

Good industrially acceptable yields are obtained only if the quantity of D-phenylglycine methylester in the reaction is in the ratio of between 4 and 6 moles per mole of 6-aminopenicillanic or 7-aminocephalosporanic acid used.

However in this case there is not only an unacceptable cost increase (the price of D-phenylglycine methyl ester is very high), but in addition by-products form which are difficult or impossible to eliminate from the desired final penicillins or cephalosporins of the reaction.

Attempts have been made using different D-phenylglycine esters or esters of different amino acids. However all these attempts have failed.

Equally negative results have been obtained using various reactive amino acid derivatives.

German patent application DOS 2214444 reports enzymatic synthesis of cephalosporins (in particular cephalexin) by reacting 7-ADCA with phenylglycine amide in the presence of the enzyme penicillin acylase. The yields obtained are very low.

PCT/DK91/00188 (WO92/01061) and PCT/DK92/00388 (WO93/12250) report methods for preparing β-lactam antibiotics by enzymatic acylation, according to which an amide of formula (III) 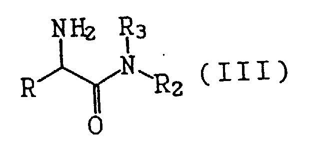 is reacted with 6-aminopenicillanic or 7-aminocephalosporanic acid in the presence of an immobilized penicillin acylase enzyme.

These methods can be implemented. It has however been found that an appreciable quantity of the amide (III) is hydrolyzed by the enzyme itself, so increasing the number of impurities in the desired final products. A small quantity of the final product is also hydrolyzed by the same enzyme.

As far back as the initial application of penicillin acylase (E.C. 3.5.1.11) it has been known that this enzyme is inhibited by the presence of phenylacetic acid, phenoxyacetic acid and/or other inhibitors, in the presence of which the enzyme loses activity and the catalysis tends to stop. This is a problem when acylating β-lactam nuclei which can contain traces of phenylacetic acid, phenoxyacetic acid or other inhibitors.

This problem has been much confronted by seeking enzymes which do not manifest this type of inhibition (see D.D.Y. Ryu et al., Biotechnology and Bioengineering 1985, vol. XXVII pages 953-960; A.M. Blinkowsky et al., Enzyme Microbial Technology 1993, vol. 15, pages 965-973). Enzymes were selected (often known as ampicillin acylase or amidase, cephalosporin acylase or amidase, cephalexinsynthesizing enzyme etc.) originating from various microbial sources (Acetobacter spp., Xanthomonas spp.). However, none of these enzymes is available commercially, and neither are they prepared on a large scale, as would be necessary for industrial use. This approach is therefore disfavoured by the fact of requiring considerable investment in research to perfect a specific catalyst for the synthesis of β-lactam antibiotics.

Another method of overcoming enzyme inhibition is to eliminate said inhibitors before effecting the enzymatic acylation, for example by extraction with organic solvent as described in PCT/DK91/00188. The main drawback of this method is precisely the use of organic solvents. As enzymatic acylation of the β-lactam nuclei is introduced precisely to dispense with the use of said solvents and hence achieve environmentally friendly processes, it follows that this approach has as yet not given satisfactory results.

The WO-A-95 34675 discloses and concerns a method for producing β--lactam antibiotics by enzymatic acylation of the parent β-lactam precursors (or intermediates), the method providing the addition of low amounts of modulators (inhibitors) to reaction mixtures containing the precursors, to reach therein low concentrations of from about 0,2 to 100.000 µM as described in the Examples and as it is clearly specified at the bottom of page 4 of such reference where there is written that "a too high concentration of the modulator will prevent the desired reaction to take place. Under all circumstances the useful concentration of the modulator in the reaction mixture is ....... preferably lower than 100 mM".

Since in the unpurified solutions in which the β-lactam precursors are produced by enzymatic hydrolysis the modulators are present in amounts which are from 10 to 100 times higher - even up to 300.000 µM - than those specified in the WO-A-95 34675, it necessarily means that the precursors acylated according to the method disclosed therein have been purified to a substantially pure crystalline form.

During their specific studies on reactions catalyzed by penicillin acylase, the present applicants have surprisingly discovered that the enzyme inhibition can be avoided, with resultant synthesis of β-lactam antibiotics in the presence of even large quantities of the aforementioned inhibitors. In some cases it is even possible to utilize the enzyme inhibition in terms beneficial to the synthesis, as described hereinafter, by introducing a determined quantity of an inhibitor initially not present or present only in a small quantity.

Consequently, the present invention provides an improved enzymatic process for producing penicillins and cephalosporins of formula (I) and (II) respectively, as heretofore specified, in which an unpurified 6-aminopenicillanic or 7-aminocephalosporanic acid intermediate product of formula (IV) or (V) 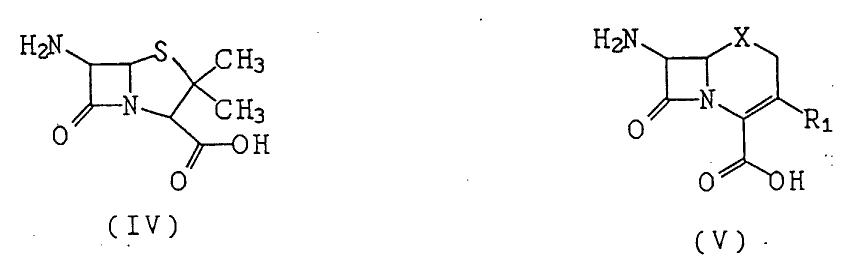 in which X and R₁ have the aforesaid meaning, is reacted at a temperature of between -5°C and +35°C, in the presence of a penicillin acylase enzyme in free or immobilized form, with an amide of formula (III) 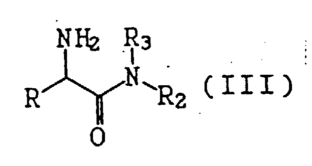 in which R has the aforesaid meaning, R₂ and R₃ are each independently a hydrogen atom or a linear or branched C₁-C₃ alkyl group, or salts thereof, in a molar ratio of between 1 and 6 moles of said amide (III) per mole of acid (IV) or (V).

With reference to formulas (I) and (II), R can be for example a phenyl group, a cyclohexadienyl group or a cyclohexanyl group either unsubstituted or substituted with a hydroxyl, a halogen, an alkyl, alkoxy, carboxyl, nitro or amino.

Ri can be a hydrogen atom, a halogen atom, a methyl group or a methylene group bound to an organic group and in particular to an alkoxy or alkoxycarbonyl group or a five or six term heterocyclic group containing from 1 to 4 heteroatoms chosen from 0, S and N bound to the methylene group by an 0, S or N atom and optionally substituted with one or more groups chosen from a hydroxyl, halogen, alkyl, alkoxy, carbonyl, carboxy, cyano, amino and the like.

The terms alkyl and alkoxy used herein refer to groups comprising from 1 to 6 carbon atoms and preferably from 1 to 4 carbon atoms.

α-amino acids having amides of formula (III) include for example D-phenylglycine, D-p-hydroxyphenylglycine and D-1,4-cyclohexadien-1-yl-glycine.

Suitable amide salts are those of an inorganic acid such as hydrochloric, hydrofluoric, hydrobromic, sulphuric or nitric acid, or of an organic acid such as acetic, formic or maleic acid.

The following acids are of particular interest for the compounds of formula (IV) and (V):
6-aminopenicillanic acid (6-APA), 7-aminodesacetoxycephalosporanic acid (7-ADCA), 7-aminocephalosporanic acid, 7-amino-3-chlorocephalosporanic acid, 7-amino-3-methoxycephalosporanic acid, 7-aminophenacetyldesacetoxycephalosporanic acid, 7-aminophenoxyacetyldesacetoxycephalosporanic acid, 7-amino-3-chlorocarbacephem, penicillin G and penicillin V.

Penicillin G or V and 7-aminophenacetyl-desacetoxycephalosporanic acid are commonly used as raw materials for preparing 6-APA and 7-ADCA respectively, these latter being used to prepare ampicillin, amoxycillin, cephalexin and cefadroxil. Up to the present time it has been necessary to eliminate the phenylacetic or phenoxyacetic acid from these intermediates (6-APA and 7-ADCA) in order to be able to use them in enzymatic acylation reactions, as described in PCT/DK91/00188. To be able to synthesize these compounds with inhibitors present means that the synthesis can advantageously proceed without the need to purify the intermediates, resulting in considerable process saving and avoiding the use of organic solvents.

The enzyme inhibitor can either be present in the reaction mixture from the commencement of synthesis or can be added at any moment during the course of the reaction. Said inhibitor can also be added at the end of the enzymatic reaction to facilitate product isolation and the subsequent processing in general.

The enzyme used as catalyst in the present process is a penicillin amidohydrolase classified as E.C. 3.5.1.11 (also known as penicillin acylase, penicillin hydrolase, ampicillin acylase etc.) originating from any microbial source and in particular from Xanthomonas, Pseudomonas, Arthrobacter, Kluyvera, Acetobacter, Escherichia, Bacillus or Acromonas strains. Enzymes deriving from natural or engineered strains of Escherichia coli are particularly preferred. Some of these are available commercially in large quantity and in immobilized form.

The enzyme can be used in free form (ie soluble) or can be immobilized on a solid matrix. Of these latter, those supports expressly dedicated to the immobilization of biomolecules, such as synthetic epoxy or azlactone resins, are particularly preferred.

### EXAMPLE 1

### Synthesis of cephalexin by enzymatic acylation of 7-ADCA with D(-)phenylglycinamide

9 g (42 mmoles) of 7-aminodesacetoxycephalosporanic acid and 15.7 g (100 mmoles) of D(-)phenylglycinamide were dissolved in water (final volume 300 ml). The solution was cooled to about 4°C and adjusted to pH 6.8, then transferred into a refrigerated reactor connected to a control system (known hereinafter as the pH-stat) maintaining the pH constant by automatically adding 4 N sulphuric acid.

5400 IU of penicillin amidohydrolase enzyme in immobilized form (on azlactone or epoxy resin) were added to the aforesaid solution, incubating at a constant 4°C and pH 6.8.

After about 75 minutes the enzyme was separated from the solution by filtration. About 4 g of β-naphthol dissolved in dilute caustic soda solution were added to the solution. 16.6 g of product in the form of cephalexin/β-naphthol complex were recovered with a molar yield on the starting nucleus of 74%. High-quality cephalexin monohydrate was then obtained (by known procedures) from the β-naphthol complex.

### EXAMPLE 2

### Synthesis of cephalexin in the presence of phenylacetic acid

17 g (0.077 moles) of impure 7-aminodesacetoxycephalosporanic acid containing as impurities 1.18% of phenylacetic acid and 1.63% of 7-aminophenacetyl-desacetoxycephalosporanic acid, and 42.68 g (0.28 moles) of D(-)phenylglycinamide were dissolved in water to a final volume of 600 ml.

The pH-stat was set to pH 7.6 and the thermostat to 2°C, 7560 IU of immobilized enzyme were used and the reaction was then conducted as described in Example 1. The reaction was interrupted after 5 hours 30 minutes and the penicillin acylase filtered off. The product was precipitated with 6 g of β-naphthol obtaining a molar yield of 87.5%. Cephalexin monohydrate was then obtained as described in Example 1.

### EXAMPLE 3

### Synthesis of cephalexin from 7 -aminophenacetyl-desacetoxycephalosporanic acid and D(-)phenylglycinamide

16 g (0.048 moles) of 7-aminophenacetyl-desacetoxycephalosporanic acid were dissolved in water adding sufficient dilute NaOH to reach pH 8.0, after which the solution was heated to 28°C and diluted with water to a final volume of 320 ml, and then transferred into a suitable reactor connected to a pH-stat and immersed in a temperature-controlled bath.

About 14000 IU of penicillin acylase enzyme in immobilized form were added to this solution (solution A), then maintaining constant pH (by adding 2N NaOH) and constant temperature.

An aqueous solution of D(-)phenylglycinamide at a concentration of about 180 g/l (solution B) was prepared separately.

After about two hours of reaction, about 190 ml of solution B were added to solution A, correcting the pH and temperature if necessary. The pH was maintained constant by adding 4N sulphuric acid.

After seven hours the enzyme was removed by filtration under vacuum. Cephalexin was recovered from the resultant solution by precipitating the cephalexin/β-naphthol complex in a quantity corresponding to a molar yield of 80.8%. Cephalexin monohydrate of quality sufficient for marketing was recovered from the cephalexin/β-naphthol complex by known procedures.

### EXAMPLE 4

### Use of inhibitors in the enzymatic acylation of 7-amino-3-chlorocephalosporanic acid

The reaction was conducted as in Example 1, but using 9.85 g (0.042 moles) of 7-amino-3-chlorocephalosporanic acid. On termination of the reaction 1.5 g of phenylacetic acid dissolved in dilute NaOH were added, after which cefaclor/β-naphthol complex was precipitated as already described in Examples 1-3 in relation to cephalexin.

The molar yield on the 7-amino-3-chlorocephalosporanic acid was 70%. Cefaclor monohydrate was obtained by decomposition of the cefaclor/β-naphthol complex (by known methods).

### EXAMPLE 5

### Enzymatic acylation of 6-aminopenicillanic acid in the presence of inhibitors

6-aminopenicillanic acid (known hereinafter as 6-APA) was obtained from penicillin G by enzymatic hydrolysis using known methods, and was isolated from the aqueous solution by precipitation with acids. The product obtained in this manner contains phenylacetic acid impurities.

20.8 g of moist impure 6-APA of 80% strength (0.077 moles) were dissolved in water and mixed with D(-)phenylglycinamide as described in Example 2, to obtain a solution containing about 0.7 g/l of phenylacetic acid. 6670 units of penicillin acylase were added and the reaction was conducted as described in Example 2. A 6-APA conversion of 82.4% was obtained.

### EXAMPLE 6

### Hydrolytic inhibition of penicillin G acylase during cephalexin synthesis in the presence of phenylacetic acid

13 g (60.75 mmoles) of 7-aminodesacetoxycephalosporanic acid (7-ADCA), 46.5 g (230.84 mmoles) of D-phenylglycine methylester.HCl and 140 mg (1.03 mmoles) of phenylacetic acid were dissolved in water to a final volume of 1000 ml.

The acylation was conducted with 17.5 g (3000 IU) of penicillin G acylase, the conditions being an initial pH of 7.25 and a temperature of 3°C. After about 3 hours 93% of 7-ADCA had been converted into cephalexin.

After the 93% conversion of 7-ADCA into cephalexin, the molar ratio of synthetic cephalexin production to hydrolytic D-phenylglycine formation was 2.15 compared with 1.44 obtained without inhibitor.

## Claims

1. A process for preparing penicillin or cephalosporin of formula (I) or (II) 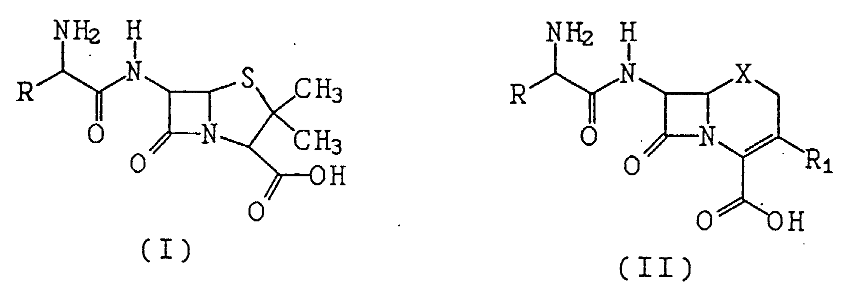 in which X is S or CH₂, R is a 6-term hydrocarbon ring optionally substituted, and R₁ is a hydrogen atom, a halogen atom, a methyl group, a methoxy group, a C₁-C₄ alkenyl group or a methylene group bound to an organic radical by an oxygen, sulphur or nitrogen atom, characterized in that an unpurified 6-aminopenicillanic or 7-aminocephalosporanic acid intermediate product of formula (IV) or (V) 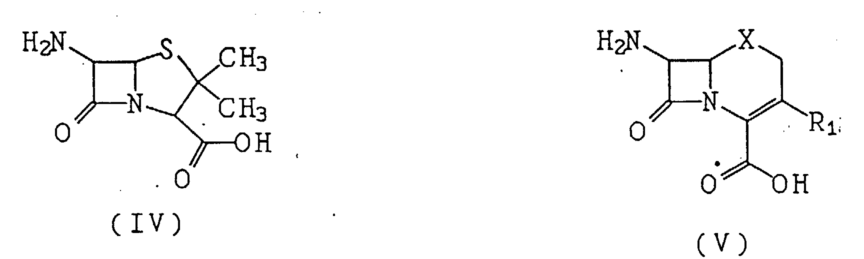 in which X and R₁ have the aforesaid meaning, is reacted in an aqueous medium at a temperature of between -5°C and +35°C, in the presence of a penicillin acylase enzyme in free or immobilized form, with an amide of formula (III) 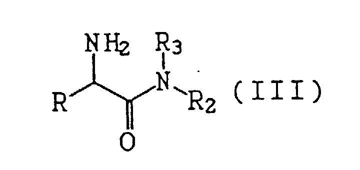 in which R has the aforesaid meaning, R₂ and R₃ are each independently a hydrogen atom or a linear or branched C₁-C₃ alkyl group, or salts thereof, in a molar ratio of between 1 and 6 moles of amide (III) per mole of acid (IV) or (V).

2. A process as claimed in claim 1, characterised in that said amide (III) is chosen from the group consisting of D-phenylglycine and its salts with an organic or inorganic acid.

## Patentansprüche

1. Verfahren zur Herstellung von Penicillin oder Cephalosporin der Formel (I) oder (II) 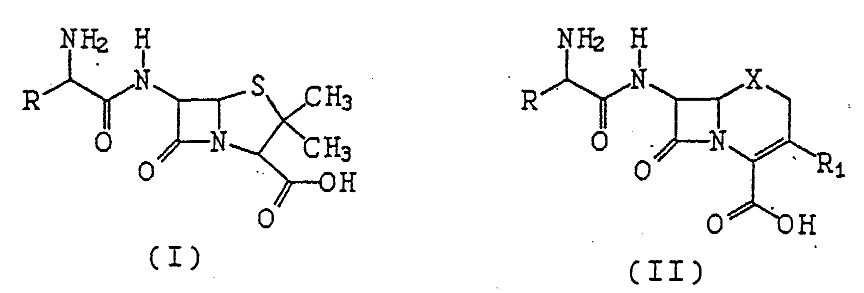 wobei X für S oder CH₂, R für einen gegebenenfalls substituierten 6-gliedrigen Kohlenwasserstoffring und R₁ für ein Wasserstoffatom, ein Halogenatom, eine Methylgruppe, eine Methoxygruppe, eine C₁-C₄-Alkenylgruppe oder eine Methylengruppe, die über ein Sauerstoff-, Schwefel- oder Stickstoffatom an einen organischen Rest gebunden ist, stehen, dadurch **gekennzeichnet**, daß ein ungereinigtes 6-Aminopenicillan-Säure- oder 7-Aminocephalosporin-Säure-Zwischenprodukt der Formel (IV) oder (V) 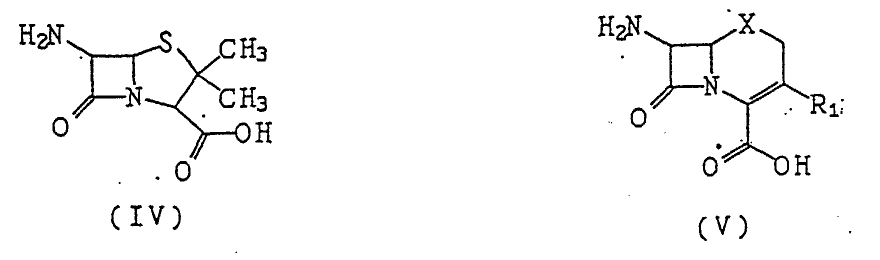 bei denen X und R₁ die vorstehende Bedeutung besitzen, in wäßrigem Medium bei einer Temperatur zwischen -5°C und +35°C in Gegenwart eines Penicillinacylaseenzyms in freier oder immobilisierter Form mit einem Amid der Formel (III) 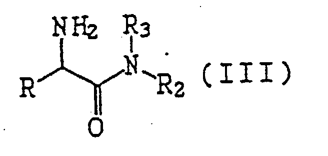 wobei R die vorstehende Bedeutung besitzt und R₂ und R₃ jeweils unabhängig voneinander für ein Wasserstoffatom oder eine lineare oder verzweigte C₁-C₃-Alkylgruppe oder Salzen davon stehen, in einem molaren Verhältnis zwischen 1 und 6 Molen des Amids (III) pro Mol der Säure (IV) oder (V) umgesetzt werden.

2. Verfahren nach Anspruch 1, dadurch **gekennzeichnet**, daß das Amid (III) aus der Gruppe bestehend aus D-Phenylglycin und dessen Salzen mit einer organischen oder anorganischen Säure ausgewählt wird.

## Revendications

1. Procédé de préparation d'une pénicilline ou d'une céphalosporine de formule (I) ou (II) 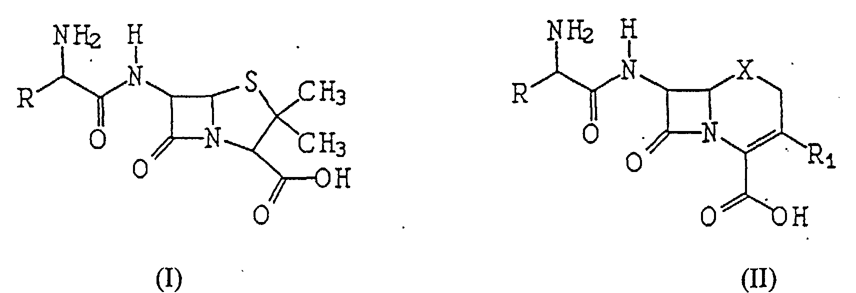 dans laquelle X est S ou CH₂, R est un noyau hydrocarboné à six chaînons éventuellement substitué, et R₁ est un atome d'hydrogène, un atome d'halogène, un groupe méthyle, un groupe méthoxy, un groupe alcényle en C₁ à C₄ ou un groupe méthylène lié à un radical organique par un atome d'oxygène, de soufre ou d'azote, caractérisé en ce qu'on fait réagir un produit intermédiaire non purifié de l'acide 6-aminopénicillanique ou de l'acide 7-aminocéphalosporanique de formule (IV) ou (V) 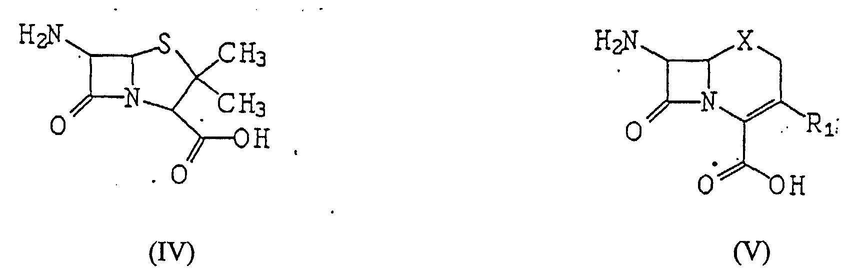 dans laquelle X et R₁ ont les significations ci-dessus, dans un milieu aqueux à une température de -5 à +35°C en présence d'une enzyme pénicilline-acylase sous une forme libre ou immobilisée, avec un amide de formule (III) 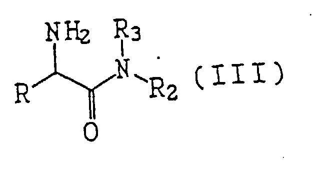 dans laquelle R a les significations ci-dessus, R₂ et R₃ représentent chacun indépendamment de l'autre un atome d'hydrogène ou un groupe alkyle en C₁ à C₃ à chaîne droite ou ramifiée, ou leurs sels, selon un rapport en moles compris entre 1 et 6 moles de l'amide (III) par mole de l'acide (IV) ou (V).

2. Procédé selon la revendication 1, caractérisé en ce que ledit amide (III) est choisi dans l'ensemble comprenant la D-phénylglycine et ses sels avec un acide organique ou inorganique.
